# EUROPEAN PATENT APPLICATION

(11) **EP 3 909 618 A1**
(43) Date of publication of application: **17.11.2021**
(21) Application number: 21173756.4
(22) Date of filing: 13.05.2021
(51) Int. Cl.: A61L 9/20

(54) **AIR SANITISING DEVICE**

(30) Priority: 14.05.2020 IT 202000011125
(71) Applicant: Nippon Gases Pharma S.r.l., 20159 Milano (IT)
(72) Inventor: GATTI, Luca, 20148 Milano (IT); LESMA, Gianluca, 20091 Bresso (Milano) (IT)
(74) Representative: Petruzziello, Aldo

(57) **Abstract**

Device (1) for sanitising air in a room (10), comprising a body (2) having a lower inlet opening (3) and an upper outlet opening (4) of a flow of air (5), ventilation means (6) for generating said flow of air, means (7) for sanitising said flow of air and means (8) for filtering said flow of air, wherein said filtering means (8) comprise three filters in series: a first active carbons filter (20) co-operating with a second glass microfibre filter (21) co-operating with a third electrostatic filter (22), said first filter (20) and said second filter (21) being placed between said ventilation means (6) and said upper outlet opening (4), while said third filter (22) is placed between said ventilation means (6) and said lower inlet opening (3).

## Description

The present invention relates to a device for sanitising air, more particularly for closed environments with high footfall.

The air we breathe is a decisive factor for our health, for our well-being and our productivity.

Contrary to possible expectations, pollution is not a phenomenon that solely relates to the air we breathe on the streets of our cities, but also involves the air we breathe in our homes, offices and schools. In other words, in all those closed environments where we spend up to 90% of our time.

Furthermore, indoor air can be more polluted than outdoor air. The EPA (Environmental Protection Agency - USA), through the IEMB (Indoor Environment Management Branch), has shown that concentrations of pollutants in indoor air are generally 2 to 5 times higher than outdoor air.

In addition to pollutants coming and trapped from the outside, indoor air can also in fact contain other internally generated pollutants. These are mainly biological pollutants and chemical-physical pollutants:
- Microorganisms: fungi, bacteria, viruses, parasites, protozoa. Their presence in indoor air represents a potential source of transmission of some infectious diseases of an epidemic nature.
- Allergens: dust mites, pollen, animal-derived allergens.
- Moulds: their presence can be linked to high humidity and inadequate ventilation and they represent one of the main causes of allergic reactions such as asthma, conjunctivitis, rhinitis and dermatitis.
- Combustion gases (NOx, SO2, CO)
- airborne atmospheric particulate matter
- VOCs, volatile organic compounds
- formaldehyde
- passive smoking from tobacco combustion
- PAHs, polycyclic aromatic hydrocarbons.

These agents can come from outside or be generated internally.

High exposure to these pollutants can have effects on the respiratory system, causing allergies and asthma, or immune system disorders.

Among the various measures to reduce pollution in closed environments, the simplest and most immediate action is undoubtedly that of opening the windows to allow a change of air. Ventilation, which is recommended at least 2/3 times a day for five minutes, not only reduces the concentration of biological pollutants but also controls temperature and humidity.

Manual ventilation allows the change of a large flow of air but does not guarantee a uniform flow of air in the rooms and therefore good air quality. For this reason, mechanical ventilation is often used.

Monitoring the concentration of pollutants in closed environments with high footfall is extremely important in order to protect people's health and well-being.

Change of air with manual or mechanical ventilation is not always sufficient for guaranteeing an adequate level of air quality.

The task set by the present invention is to eliminate the above-mentioned disadvantages of the prior art.

Within the scope of this task, an object of the invention is to produce an air sanitising device which allows all the polluting substances, in particular bacteria, viruses, moulds, particulate matter and VOCs (Volatile Organic Compounds), in rooms to be destroyed.

It is further an object of the invention to create an air sanitising device that allows safe sanitisation of the interior of a room and which can be easily transported according to needs from one room to the other by any person without the need for any type of particular tools for this purpose.

Last but not least, the object of the invention is to create an air sanitising device which allows anyone to be able to read the air quality and the state of the filters at any time and which can provide further information about the same and the surrounding environment should this be required.

This task as well as these and other objects are achieved by a device for sanitising the air in a room, comprising a body having a lower inlet opening and an upper outlet opening of a flow of air, ventilation means for generating said flow of air, means of sanitising said flow of air and means of filtering said flow of air, wherein said filtering means preferably comprise three filters in series: a first active carbons filter co-operating with a second filter in glass microfibre co-operating with a third electrostatic filter. In a constructional variant, said filtering means comprise two filters in series: a first HEPA (High-Efficiency Particulate Air) filter and a second electrostatic filter.

Conveniently, said first filter and said second filter are placed between said ventilation means and said upper outlet opening, while said third filter is placed between said ventilation means and said lower inlet opening.

Advantageously, said filters are mounted on respective extractable frames.

These features and advantages of the invention will be made clearer by the following description by a non-limiting example of preferred but not exclusive embodiments of an air sanitising device illustrated in the accompanying drawings, wherein:

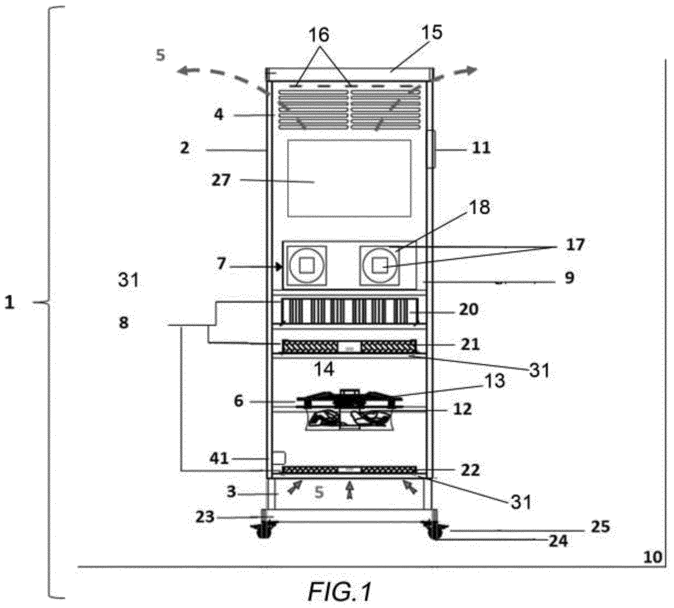

- Fig. 1 is a partially sectioned front elevation view of the air sanitising device according to the invention;

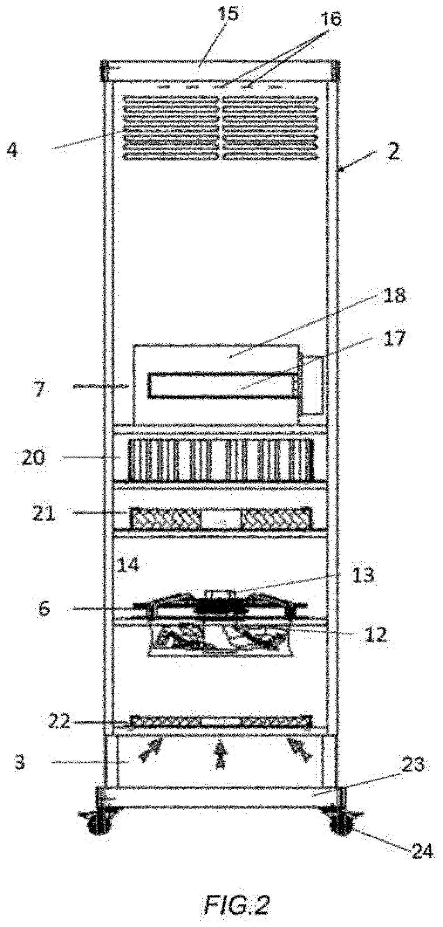

- Fig. 2 is a partially sectioned side elevation view of the air sanitising device according to the invention;

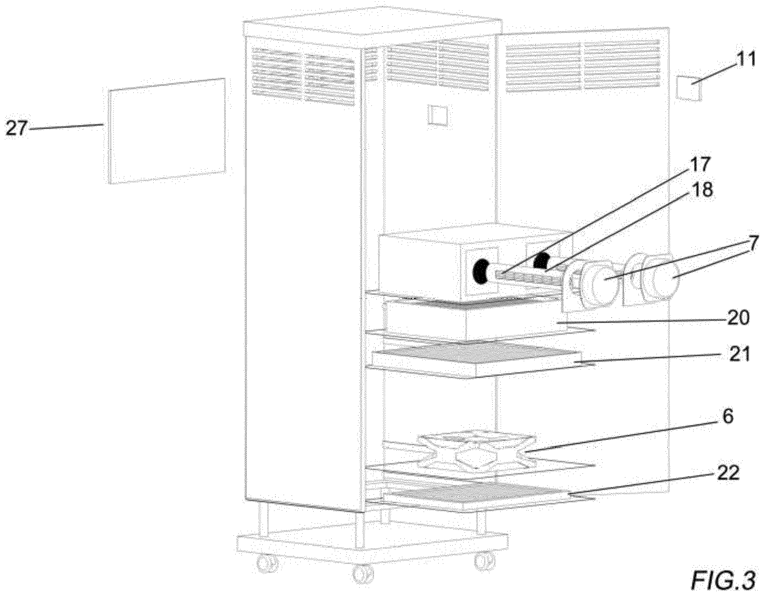

- Fig. 3 is an axonometric view of the device for the sanitisation of air of the preceding drawings, with the front door open and the main components shown in blown-up view.

Referring in particular to the drawings described above, the air sanitising device according to the invention is generically denoted by reference numeral (1). The air sanitising device (1) is preferably used in environments where several people gather together such as, for example, a room (10) or a waiting room of a public/private building, of a hospital or a doctor's office and in all those closed places where a good air quality is required.

The device (1) comprises a body (2) having a lower inlet opening (3) with a grille and an upper outlet opening (4) with grille for a flow of air (5).

The inlet opening has a metal mesh to prevent the passage of bodies of considerable dimensions and a plate with a plurality of holes designed both to prevent the passage of bodies of small dimensions and to evenly distribute the flow of air (5); the outlet opening has an MDF wood grille and a metal mesh to protect the interior of the body.

Inside the body (2) ventilation means (6) are present for generating a flow of air that can in a short time sanitise the entire air of the room or area where the device is placed.

In the body (2) are present, moreover, means for sanitising (7) and filtering (8) the flow of air.

The body (2) is made in MDF wood panels and, in a preferred but not exclusive embodiment, has a parallelepiped conformation.

Advantageously, a viewer (11) is present on a side face of the body (2) to indicate graphically or numerically the air quality present in the room.

The viewer (11) may be of the touch screen type or may be controlled from a keypad inside the body (2) or remotely from a remote location.

The ventilation means (6) comprise at least one tangential fan (12), with multiple rotation speeds, actuated by an electric motor (13) and housed inside a sealed channel (14) which develops coaxially inside the parallelepiped body (2).

This solution allows the flow of air to be aspirated with few load losses from the inlet opening (3) and sent sanitised to the outlet opening (5).

An upper tile (15), acting as a closure, ensures that the air is directed into the room substantially orthogonal to the axis of the body (2) over 360°. In one constructional variant, the air is directed upwards through the upper tile.

LEDs (16) placed at the outlet opening (4) have a dual function: to illuminate the outlet opening and in general to indicate other parameters relating to the functioning of the device.

The sanitising means (7) include at least one lamp (17) for activating a catalyst (18) for sanitising the flow of air housed in the channel (14) and placed between the fan and the outlet opening and extractable by means of an extractable frame (31).

Advantageously, the lamp (17) is an ultraviolet lamp of the high intensity type and the catalyst (18) is made in a metal alloy with a honeycomb matrix made up of titanium dioxide and other noble metals.

More particularly, the lamp being high intensity has technical features determined by UV light with a wavelength of less than 385 nanometres able to optimally activate the catalyst which is placed at a predetermined distance therefrom as a function inherently of its technical and functional features.

In addition to titanium dioxide, the metal alloy of the catalyst contains rhodium for the conversion of the oxides, silver and copper for the acceleration of the reaction process and platinum for attracting and retaining the electrons, delaying the recombination thereof.

In a constructional solution, the lamp is placed with its longitudinal axis perpendicular to the axis of the body (2) and the catalyst is placed adjacent thereto.

In a constructional variant, the lamp can be placed coaxially to the body (2) and the catalyst partially or totally surrounds it.

The sanitising means carry out photocatalysis which enables the pollutants, in particular bacteria, viruses, moulds and odours, to be destroyed with a natural active ingredient.

More particularly, the sanitising means generate a photochemical reaction that binds an additional oxygen molecule (O) to the pre-existing hydrogen and oxygen ones of the moisture present in the air (H₂O), thus generating both hydrogen peroxide (H₂O₂) and oxidising ions (OH-).

Hydrogen peroxide (H₂O₂ and the oxidising ions (OH-) generated by the reaction possess very high efficacy in destroying the microbial load, both in the air and on the surfaces of the environment treated and on the objects contained therein.

The filtering means comprise a first active carbons filter (20) co-operating with a second compact rigid pocket filter with low load loss in glass microfibre paper (21) co-operating with a third electrostatic washable filter in polypropylene with alternating polarities (22). According to a construction variant, the first active carbons filter (20) and the second glass microfibre filter (21) are replaced by a HEPA (High-Efficiency Particulate Air) filter.

The third filter (22) moreover has a steel mesh, not shown, which prevents deformation thereof even in the case wherein the speed of the fan is increased to increase the flow of air to be sanitised.

In this way the subsequent second filter (21) and first filter (20) are protected from deformation, breakages and considerable variations in filtration efficiency, in that the third air filter (22) also functions as a pressure compensator, keeping it substantially within optimal working values on the two subsequent filters.

In order to better optimise the pressures inside the housing (2) a pressure switch (41) is also present.

In a preferred solution the first filter and the second filter are placed between the fan and the outlet opening (4) in such a way that the fan (12) and the motor (13) can be lowered towards the inlet opening (3) lowering the centre of gravity of the body (2) so as to make it more stable both when it is transported and when it is positioned in the room.

Appropriately, the third filter (22) is housed in the channel (14) between the fan (12) and the inlet opening (3).

In this way a progression of the retention of all the polluting particles present in the aspirated air is obtained, obtaining two immediate results.

The first result is that the fan blades (12) and the channel (14) itself are prevented from becoming dirty during functioning and unexpectedly and suddenly releasing a high number of polluting particles in the flow of air to be sanitised.

The second result which is obtained is the progressive optimisation of the sanitisation of the flow of air at all levels of type and size of the harmful particles present therein.

For this reason, the three filters are set to work in series at a distance proportional to their amplitude in order to progressively optimise the individual work of each thereof.

However, this does not prevent all the filters from being housed below or above the ventilation means (6).

Each filter, moreover, is mounted on a frame (31) that can be extracted from the channel (14) by means of sliding guides, which allow easy control and/or replacement thereof after predetermined periods of functioning, which are indicated on each occasion on the viewer (11).

The inlet opening (3) comprises a base (23) which allows aspiration of the flow of air over 360° and which has pivoting wheels (24) provided with a locking system (25) in order to easily move the device from one room to the other and keep it in a fixed position inside the same.

The body (2) comprises a rear door of opening and closure with lock, and a housing for a screen (27), which is placed internally to the body (2), on said rear door, in such a way as not to generate therein load losses due to obstructions.

For this reason, in fact, the fan (12) expels the flow of air through a mouth of small dimensions and having a plurality of holes designed to optimise the pressures acting on the filter above it (21).

The screen (27) is visible frontally from the outside, through a portion of transparent front wall of the device and carries advertising and information in general.

The preferred dimensions of the sanitising device according to the invention are 193 cm x 68 cm x 58 cm.

It has been found in practice that the air sanitising device according to the invention is particularly advantageous for the elimination of germs, bacteria, viruses and the elimination of odours inside a room.

In practice, the materials used, as well as the dimensions, can be any according to requirements and the state of the art.

Naturally, the invention is not limited to the particular embodiment described above and illustrated in the accompanying drawings, but numerous detailed changes may be made thereto within the reach of the person skilled in the art, without thereby departing from the scope of the invention itself, as defined in the following claims.

## Claims

1. Device (1) for the sanitisation of air in a room (10), comprising a body (2) having a lower inlet opening (3) and an upper outlet opening (4) for a flow of air (5), ventilation means (6) for generating said flow of air, means (7) for sanitising said flow of air and means (8) for filtering said flow of air, **characterised in that** said filtering means (8) comprise three filters in series: a first active carbons filter (20) co-operating with a second glass microfibre filter (21) co-operating with a third electrostatic filter (22), said first active carbons filter (20) and said second glass microfibre filter (21) being placed between said ventilation means (6) and said upper outlet opening (4), while said third filter (22) is placed between said ventilation means (6) and said lower inlet opening (3).

2. Air sanitising device according to claim 1, wherein said first active carbons filter (20) and said second glass microfibre filter (21) are replaced by a HEPA (High-Efficiency Particulate Air) filter.

3. Air sanitising device according to claim 1 or 2, **characterised in that** said filters (20, 21, 22) are mounted on respective extractable frames (31).

4. Air sanitising device according to any one of the preceding claims, **characterised in that** said ventilation means comprise at least one fan (12) actuated by an electric motor (13) designed to aspirate said flow of air from said inlet opening (3) and send it to said outlet opening (4).

5. Air sanitising device according to any one of the preceding claims, **characterised in that** said sanitising means are arranged between said ventilation means (6) and said outlet opening (4) and comprise at least one lamp (17) for activation of a catalyst (18) for the sanitisation of said flow of air.

6. Air sanitising device according to any one of the preceding claims, wherein said inlet (3) and outlet (4) openings are provided with respective grilles and allow, respectively, aspiration and expulsion of air over 360°.

7. Air sanitising device according to any one of the preceding claims, **characterised in that** a viewer (11) is present on a face (9) of said body (2) suitable for indicating at least the quality of the air present in said room (10).

8. Air sanitising device according to any one of the preceding claims, **characterised in that** said body comprises a rear opening and closure door, and a housing for a screen (27), placed inside said body (2) and visible frontally from the exterior.

9. Air sanitising device according to any one of the preceding claims, **characterised in that** at said outlet opening (4), LEDs (16) are provided for at least its lighting and, by taking on different colours, to indicate parameters related to the functioning of the device, such as the state of sanitisation of the flow of air and/or the state of the quality of the air inside the room.

10. Air sanitising device according to any one of the preceding claims, **characterised in that** said ventilation means (6), said sanitising means (7) and said filtering means (8) are housed in a sealed channel (14) inside said body (2).

11. Air sanitising device according to claim (1), **characterised in that** said body (2) is made of MDF wood panels, has a parallelepiped shape and rests on a base with pivoting wheels (24) equipped with a locking system (25).
